# EUROPEAN PATENT APPLICATION

(11) **EP 2 353 620 A1**
(43) Date of publication of application: **10.08.2011**
(21) Application number: 09823328.1
(22) Date of filing: 29.10.2009
(51) Int. Cl.: A61L 27/00, A61F 2/14, A61F 2/16

(54) **OPHTHALMIC COMPOSITION CAPABLE OF FORMING GEL**

(30) Priority: 31.10.2008 JP 2008280742
(71) Applicant: Hoya Corporation, Tokyo 161-8525 (JP); Public University Corporation Nara Medical University, Nara 634-8521 (JP); Kyushu University, National University Corporation, Fukuoka-shi Fukuoka 812-8581 (JP)
(72) Inventor: YOKOYAMA, Yuichi, Tokyo 161-8525 (JP); MATSUURA, Toyoaki, Kashihara-shi Nara 634-8521 (JP); HARA, Yoshiaki, Kashihara-shi Nara 634-8521 (JP); ANNAKA, Masahiko, Fukuoka-shi Fukuoka 812-8581 (JP); MARUYAMA, Keiichi, Kawasaki-shi Kanagawa 210-0865 (JP); TANAKA, Susumu, Kawasaki-shi Kanagawa 210-0865 (JP)
(74) Representative: von Kreisler Selting Werner
(86) International application number: PCT/JP2009/005729
(87) International publication number: WO 2010/050215

(57) **Abstract**

Disclosed is a material for use in ophthalmologic organ replacement, which is transparent, has controllable physical properties, and can have physical properties suitable for use as an ophthalmic tissue when the hardness of a gel produced from the material or the fluidability of a sol produced from the material at a specific temperature or lower is varied. Specifically disclosed is an ophthalmologic composition capable of forming a gel, which comprises a polyethylene glycol that is represented by formula 1 and has one end modified with a long-chain alkyl group, a polyethylene glycol that is represented by formula 2 and has both ends modified with a long-chain alkyl group, and a solvent.

R₁-O-(CH₂CH₂O-)n1-R₂ (Formula 1)

[In formula 1, R₁ represents an alkyl group having 1 to 4 carbon atoms; R₂ represents an alkyl group having 16 to 22 carbon atoms; and n1 represents the average number of moles of added oxyethylene groups and falls within the range from 45 to 450.]

R₃-O-(-CH₂CH₂O-)n2-R₄ (Formula 2)

[In formula 2, R₃ and R₄ independently represents an alkyl group having 16 to 22 carbon atoms; and n2 represents average number of moles of added oxyethylene groups and falls within the range from 45 to 450.]

## Description

### Cross-reference to related patent application

The present application claims priority under Japanese Patent Application 2008-280742, filed on October 31, 2008, the entire contents of which are hereby incorporated by reference.

### [Technical Field]

The present invention relates to an ophthalmologic composition having gelation ability that contains modified polyethylene glycol. More particularly, the composition of the present invention is a composition for replacing ophthalmologic organs that is useful as a vitreous body substitute, particularly as a vitreous body substitute, or an artificial vitreous body, having a tamponade effect.

### [Background Art]

The vitreous body is a jelly-like substance comprised of collagen and sodium hyaluronate that fills the posterior cavity of the eye. The vitreous body is a substance within the eye that is important not only in terms of its optical and dynamic characteristics, but also in terms of its effects on peripheral tissue, the overall condition of the eye, and disease. In humans, sight is an important means of gathering information. Thus, various treatment methods have been devised. For example, proliferative diabetic retinopathy and rhegmatogenous retinal detachment, previously considered untreatable, can now be treated. Among these treatments, invasive surgery, the treatment of first choice, removes the vitreous body. Thus, replacement materials and materials with tamponade effects have been researched and developed, and their clinical applications have been examined.

Polyethylene glycol, polyvinyl alcohol, collagen, sodium hyaluronate, acrylic acid-synthesized polymeric materials, and the like have been examined as substitute materials for the vitreous body. However, polyvinyl alcohol is a crystalline polymer and thus undergoes a change in volume upon recrystallization, tends to clouds due to birefringence, and the like. For such reasons, its clinical application is impractical. Collagen and sodium hyaluronate are expensive due to their biological/microbiological derivation, are problematic in terms of viscosity control, and difficult to handle during injection, rendering their clinical application impractical. Polymers synthesized from acrylic acid are not actually being employed clinically for reasons such as the toxicity of their monomer components. Low-molecular-weight lipophilic pharmaceutical implant compositions that are derivatives of glycerol and having sol-gel transition points in the vicinity of the temperature of the human eye have also been disclosed. Due to low molecular weight, when transplanted into the eye, they end up passing through eye tissue and being discharged outside the eye, rendering them unsuitable as long-term implant substitute materials and materials having tamponade effects (Patent Reference 1).

Silicone oils, perfluorocarbons, and the like are currently being clinically employed as temporary substitutes for the vitreous body that have tamponade effects. However, silicone oils are difficult to use for reasons such as toxicity to eye tissue, repeat surgery to remove films caused by repeat proliferation of preretinal proliferative membranes, emulsification clouding relating to quality, and the like. They are currently employed as a countermeasure in cases of intractable retinal detachment (Patent References 2 and 3).

Currently, liquid perfluorocarbons are employed as temporary tamponades; air is employed as a gas tamponade; and sulfur hexafluoride (SF6), propane octafluoride, and the like are employed as expansion gases. Liquid perfluorocarbons produce tamponade effects based on their specific gravities. They thus present problems such as subretinal invasion and aberration in the anterior chamber in aphakia and pseudophakia. In gas tamponade, absorption of the gas within the eye limits the effect to from one day to one week. Further, when the gas pressure is elevated, glaucoma and the like are a concern due to high eye pressure. Because a gas is involves, difficulty in peering through to the optical fundus following surgery due to clouding, and the like, of the posterior capsule, toxicity to eye tissue, and a rise in eye pressure are concerns (Patent Reference 3).

As set forth above, the air, hyaluronan, silicone oil, perfluorcarbons, and the like that are currently in use are less than ideal due to problems with biocompatibility, service lifetime, handling, and the like.
[Patent Document 1] JP-A-2006-500328
[Patent Document 2] WO2004/026953
[Patent Document 3] JP-A-H06-154263
The entire contents of Patent Documents 1-3 are hereby incorporated by reference.

In light of the above circumstances, the present invention provides a new transparent material with physical properties that can be controlled, and in particular, a material for ophthalmologic organ replacement having physical properties suited to eye tissue achieved by varying the gel toughness and the fluidity of the sol at certain temperatures.

### [Summary of the Invention]

As the result of extensive research into solving the above problems, it was found that by adjusting, in a solution comprised of a mixture of polyethylene glycol modified on both ends with long-chain alkyl groups ("di-PEG" hereinafter) and PEG modified on one end with a long-chain alkyl group ("mono-PEG" hereinafter), the blending ratio of di-PEG to mono-PEG, it was possible to vary the gel toughness and vary the fluidity of the sol at certain temperatures. As a result, since physical properties suited to eye tissue were achieved, the material was discovered to be suited to ophthalmologic organ replacement. The present invention was devised on that basis.

The present invention relates to an ophthalmologic composition having gelation ability, comprising the polyethylene glycol denoted by Formula 1, one end of which is modified with a long-chain alkyl group; the polyethylene glycol denoted by Formula 2, both ends of which are modified with long-chain alkyl groups; and a solvent:

R₁-O-(CH₂CH₂O-)ₙ₁-R₂ (Formula 1)

wherein
R₁ denotes an alkyl group with 1 to 4 carbon atoms;
R₂ denotes an alkyl group with 16 to 22 carbon atoms, and
the average number of moles of added oxyethylene groups n1 falls within a range of 45 to 450;

R₃-O-(-CH₂CH₂O-)ₙ₂-R₄ (Formula 2)

wherein
each of R₃ and R₄ independently denotes an alkyl group with 16 to 22 carbon atoms, and
the average number of moles of added oxyethylene groups n2 falls within a range of 45 to 450.

In the above composition of the present invention,
the mass ratio of the polyethylene glycol denoted by Formula 1 and the polyethylene glycol denoted by Formula 2 can fall within a range of 50 to 99:50 to 1;
the solvent can be water or physiological saline;
the combined content of the polyethylene glycol denoted by Formula 1 and the polyethylene glycol denoted by Formula 2 can fall within a range of 10 to 40 weight percent; a pH-adjusting agent can be further incorporated; and a fluorescent dye and/or coloring agent can be incorporated.

The composition of the present invention can be employed as an artificial vitreous body or artificial crystalline lens.

### [Effect of the Invention]

The present invention provides a material for ophthalmologic organ replacement having physical properties that are rendered suitable to eye tissue by changing the gel toughness and changing the fluidity of the sol at certain temperatures.

### [Brief Description of the Drawings]

Fig. 1 shows the sol-gel transition point.
Fig. 2 is a comparison of light transmittance and the vitreous body of the human eye (literature).

### [Modes of Carrying Out the Invention]

The present invention relates to an ophthalmologic composition having gelation ability, comprised of polyethylene glycol modified on one end with a long-chain alkyl group; polyethylene glycol modified on both ends with long-chain alkyl groups; and a solvent.

The polyethylene glycol (PEG) modified on one end with a long-chain alkyl group that is contained in the ophthalmologic composition having gelation ability of the present invention is denoted by Formula 1 below. The polyethylene glycol denoted by Formula 1 below will on occasion be denoted as "mono-PEG."

R₁-O-(CH₂CH₂O-)ₙ₁-R₂ (Formula 1)

In the formula, R₁ denotes an alkyl group with 1 to 4 carbon atoms; R₂ denotes an alkyl group with 16 to 22 carbon atoms, and the average number of moles of added oxyethylene groups n1 falls within a range of 45 to 450.

The PEG denoted by Formula 1 is an amphipathic polymer combining a hydrophilic moiety and a hydrophobic moiety within the molecule. In water, the amphipathic polymer condenses so that the hydrophobic groups avoid contact with water molecules, and the hydrophilic groups self- assemble so that they cover the surface of the hydrophobic groups. This state refers to a phase that loses fluidity when a large quantity of solvent is contained, has a three-dimensional network structure that is insoluble in a specific solvent, and is known as a gel. The phase having fluidity prior to becoming a gel is called a sol. The transition from sol to gel is referred to as a sol-gel transition. When the amphipathic polymer exceeds a certain critical concentration in solvent, the hydrophilic PEG chains undergo physical crosslinking, causing a sol-gel transition. The amphipathic polymer in the gelled state undergoes a sol-gel transition over a specific temperature range for each concentration. The present invention provides a substitute that can be injected into and gelled in the vitreous body cavity by utilizing these properties.

The average number of moles of added oxyethylene groups n1 suitable falls within a range of 45 to 450. That is, when the average number of moles of added oxyethylene groups n1 denoted in Formula 1 is less than 45, the gel obtained becomes excessively tough. Conversely, when 450 is exceeded, manufacturing becomes difficult. The average number of moles of added oxyethylene groups n1 desirably falls within a range of 60 to 350, preferably within a range of 70 to 180.

When the number of carbon atoms in short-chain alkyl group R₁ exceeds 4 and the composition of the present invention is gelled, it becomes excessively tough and handling such as injection into the eye becomes difficult. Thus, four or fewer carbon atoms are suitable. Specifically, the alkyl group with an integer number of carbon atoms of 1 to 4 that is denoted by R₁ can be a methyl group, ethyl group, n-propyl group, isopropyl group, n-butyl group, isobutyl group, t-butyl group, or the like. Alkyl group R₁ is optimally a methyl group with one carbon atom.

When long-chain alkyl group R₂ contains fewer than 16 carbon atoms, the composition of the present invention does not gel. When it contains more than 22 carbon atoms and the composition of the present invention is gelled, the gel is excessively tough and handling such as injection into the human eye becomes difficult. Thus, a carbon number falling within a range of 16 to 22 is suitable. The alkyl group with 16 to 22 carbon atoms that is denoted by R₂ can be linear or branched. Specific examples are a hexadecyl group, octadecyl group, eicosyl group, docosyl group, and isostearyl group. Alkyl group R₂ is optimally an octadecyl group with 18 carbon atoms.

The PEG modified at both ends with long-chain alkyl groups that is contained in the ophthalmologic composition having gelation ability of the present invention is a PEG Formula 2 that is modified at both ends with long-chain alkyl groups. The PEG denoted by Formula 2 will on occasion be denoted as "di-PEG" hereinafter.

R₃-O-(-CH₂CH₂O-)ₙ₂-R₄ (Formula 2)

In the formual, each of R₃ and R₄ independently denotes an alkyl group with 16 to 22 carbon atoms, and the average number of moles of added oxyethylene groups n2 falls within a range of 45 to 450.

The toughness of the gel formed by the composition of the present invention and the fluidity of the sol at certain temperatures can be varied by means of the blending ratio of the di-PEG denoted by Formula 2 and the mono-PEG denoted by Formula 1, providing a material for ophthalmologic organ replacement having physical properties suited to eye tissue.

The average number of moles of added oxyethylene groups n2 suitably falls within a range of 45 to 450. When the average number of added oxyethylene groups n2 of the polyethylene glycol denoted by Formula 2 is less than 45, the gel obtained is excessively tough. Conversely, at greater than 450, manufacturing becomes difficult. The average number of moles of added oxyethylene n2 desirably falls within a range of 60 to 340, and preferably falls within a range of 140 to 320.

When the number of carbon atoms of long-chain alkyl groups R₃ and R₄ is less than 16, the composition of the present invention does not gel. When the number of carbon atoms exceeds 22 and the composition of the present invention is gelled, the gel is excessively tough and handling such as injection into the eye becomes difficult. Thus, the number of carbon atoms suitably falls within a range of 16 to 22. The alkyl groups with 16 to 22 carbon atoms that are denoted by R₃ and R₄ can be linear or branched. Specific examples are hexadecyl groups, octadecyl groups, eicosyl groups, dodecyl groups, and isostearyl groups. R₃ and R₄ can be identical or different. Both R₃ and R₄ desirably denote octadecyl groups.

The mono-PEG denoted by Formula 1 and the di-PEG denoted by Formula 2 can be synthesized by introducing alkyl groups onto both ends of polyethylene glycol. A known method can be suitably employed as the synthesis method. The polyethylene glycol that is employed as a starting material can also be manufactured by known manufacturing methods. Normally, ethylene oxide is dripped in a pressurized reaction device at 40 to 250°C in the presence of an alkali catalyst to obtain polyethylene glycol that can be employed as a starting material for the end-modified polyethylene glycol having the molecular weight or average number of moles added of the above range.

The weight ratio of the mono-PEG denoted by Formula 1 and the di-PEG denoted by Formula 2 desirably falls within a range of 50 to 99:50 to 1 in the composition of the present invention. By remaining within this range, it is possible to obtain a composition having physical properties (such as gel toughness, fluidity, and handling) that are suited to eye tissue. However, the physical properties such as gel toughness, fluidity, and handling can be varied based on the types of alkyl groups present in the mono-PEG and di-PEG, the molecular weight or average number of moles of added oxyethylene groups in the mono-PEG and di-PEG, and the combined content of mono-PEG and di-PEG in the composition of the present invention. Accordingly, the weight ratio can be considered on the basis of these factors and suitably selected to impart physical properties suited to the targeted eye tissue. For example, as indicated in the embodiments, in the case of a mixture of mono-PEG where n1 is about 110, R1 is a methyl group, R2 is an octadecyl group, and di-PEG where n2 is about 250, R3 is an octadecyl group, and R4 is an octadecyl group, it is desirable for the weight ratio to fall within a range of 80 to 95:20 to 5 from the perspective of physical properties such as gel toughness, fluidity, and handling.

The composition of the present invention comprises a solvent in addition to the mono-PEG of Formula 1 and the di-PEG of Formula 2. By way of example, water or physiological saline can be employed as the solvent. Sodium chloride, potassium chloride, and the like can be employed as isotonic agents contained in physiological saline. A pH-adjusting agent in the form of an alkali metal salt of an acid such as phosphoric acid, boric acid, or ethylenediamine tetraacetic acid (EDTA) can be incorporated. A pH buffering effect can also be imparted to the composition of the present invention.

The combined content of the mono-PEG and di-PEG in the composition of the present invention suitably falls within a range of 10 to 40 weight percent. In a combined quantity exceeding 40 weight percent, the transition temperature of the mono-PEG and di-PEG is unstable, the gel formed by the composition of the present invention becomes excessively tough, and handling such as injection into the eye tends to become difficult. Conversely, when the combined content of mono-PEG and di-PEG is less than 10 weight percent, gelation tends to become difficult. However, the ability to gel of the composition of the present invention varies with the content of di-PEG (its weight ratio to the mono-PEG). The combined content of the mono-PEG and di-PEG desirably falls within a range of 15 to 30 weight percent, preferably within a range of 20 to 25 weight percent. Within a range of 20 to 25 weight percent, a gelled state can be maintained at from 35 to 40°C, which is close to the temperature of the human eye (body temperature). The combined content of mono-PEG and di-PEG is sometimes referred to as the gel concentration in the present Specification.

Fluorescent dyes and coloring agents can be incorporated into the composition of the present invention. The incorporation of fluorescent dyes and coloring agents can be used to impart visibility to the interior of the eye. The fluorescent dyes and coloring agents that are employed are preferably of relatively high molecular weight, such as pyrene, fluorescein, and uranine, from the perspective of stability of incorporation and the like into the body.

### ○ Evaluation of physical properties of gel based on content of crosslinking components

The di-PEG content (weight percent) was compounded to yield a prescribed weight when the weight of the mixture of mono-PEG and di-PEG was denoted as 100, and a solution was prepared in a heat-resistant test tube with a screw opening measuring 16 mm (dia.) x 125 mm so as to achieve a gel concentration in physiological saline of 20 percent. The cap was installed on the test tube containing the solution. The solution that had been prepared was left standing in a DP32 low-temperature vacuum drier made by Yamato Scientific Co., Ltd. that had been set to 70°C and heated overnight (for about 16 hours), yielding a solution in the sol state. When it did not dissolve at 70°C, it was heated to 80°C to obtain a solution in the sol state. The solution that had been prepared was statically positioned in an IW240 hygrostat made by Yamato Scientific Co., Ltd. in a cool incubator set to 37°C, gelation at 37°C was confirmed, and a gel was obtained for measurement of physical properties. Various tests were conducted on the physical properties of the gel based on the 37°C (near body temperature) toughness of the gel, transparency, fluidity at 70°C taking into account injection device filling properties, the time (minutes) required for gelation at 37°C of sol solutions dissolve at 70°C or 80°C, and the feel or handling in the course of injecting the gel with an injection device in the form of a 21 gauge syringe. The results and evaluation methods are given in Table 1.

The composition of the present invention exhibits a sol-gel transition point at a temperature falling within a range of 0 to 70°C that depends on the combined contents of mono-PEG and di-PEG, and on the weight ratio of the mono-PEG and di-PEG. As the content of di-PEG increases, a gelled product will become a jam-like gel. Even in a gel state, injection into the eye is possible with an injection means such as a syringe. In terms of gel fluidity, toughness, and handling, a di-PEG content of 20 weight percent or less is desirable. At greater than 20 weight percent, there is a strong tendency to give brittleness to the jam state. Since the composition of the present invention has a sol-gel transition point, even a composition with a tough, high gel concentration, that is, one with a high di-PEG content, can be packed into a syringe in a sol state if heated to 70°C or higher. Since compositions that are jam-like gels even if they are accompanied by tough brittleness, they can be readily injected into the eyeball with little invasiveness through an extremely small insertion hole after lowering their temperature to the vicinity of body temperature (37°C). From the perspective of permitting injection, the range of the di-PEG content desirably falls within a range of 1.0 to 50 percent, and from the perspective of handling and packing properties, preferably falls within a range of 1.0 to 20 percent. From the perspectives of having a sol-gel transition point close to body temperature at 35 to 40°C and good handling and packing properties, a range of 5 to 10 percent is optimal.

The ophthalmologic composition of the present invention, having such physical properties, can be employed as an artificial vitreous body and artificial crystalline lens. The ophthalmologic composition of the present invention is a composition for replacing an ophthalmologic organ that is useful as a vitreous body substitute, particularly as a vitreous body substitute and an artificial vitreous body having a tamponade effect. However, it is not limited thereto, and is also useful as a transplant material and supplemental material for eyeball tissue in the form of corneal, crystalline lens, scleral, and retinal tissue and the like. It can also be useful as a substitute for, or a transplant material or supplemental material for, the gel-like biotissue of other parts.

### [Embodiments]

The present invention is described in greater detail below through embodiments. However, the embodiments do not limit the scope of the present invention.

Polyethylene glycol derivatives denoted by Formula 1 and Formula 2 can be prepared by methods known in the field of art. Examples of the synthesis of these derivatives are given below.

### Example of synthesis of polyethylene glycol derivative mono-PEG denoted by Formula 1

### (n1: about 110; R1: methyl group; R2: octadecyl group)

To a four-necked flask equipped with nitrogen introduction tube were charged 500 g of polyethylene glycol monomethyl ether (made by NOF Corporation) in which the average number of moles of added oxyethylene groups was about 110 and 3,000 mL of toluene. While introducing dry nitrogen, the mixture was heated and dissolved. Subsequently, 20 g of sodium hydride was added in a water bath, the mixture was stirred in a nitrogen atmosphere, and the mixture was reacted for six hours at room temperature. Next, 180 g of 1-bromooctadecane (reagent guaranteed grade, made by Wako Pure Chemical Industries, Ltd.) was added and the mixture was reacted for 12 hours at 60°C in a nitrogen atmosphere with stirring. Following the reaction, 500 mL of water was added over an hour while stirring in a water bath, the product was left standing for 3 hours, and the aqueous lower layer was removed. The base number was measured, the product was neutralized by adding a quantity of dilute hydrochloric acid calculated for neutralization, the toluene was distilled off at 110°C and 6.7 KPa (50 mmHg), and the product was filtered, yielding a crude reaction product. The crude reaction product was melted by heating and then precipitated in petroleum ether by addition with stirring. The precipitate was filtered out and dried under reduced pressure to obtain the targeted product.

### Example of synthesis of polyethylene glycol derivative di-PEG denoted by Formula 2

### (n2: about 250; R3: octadecyl group; R4: octadecyl group)

To a four-necked flask equipped with nitrogen introduction tube were charged 500 g of polyethylene glycol (made by NOF Corporation) in which the average number of moles of added oxyethylene groups was about 250 and 3,000 mL of toluene. While introducing dry nitrogen, the mixture was heated and dissolved. Subsequently, 40 g of sodium hydride was added in a water bath, the mixture was stirred in a nitrogen atmosphere, and the mixture was reacted for six hours at room temperature. Next, 360 g of 1-bromooctadecane (reagent guaranteed grade, made by Wako Pure Chemical Industries, Ltd.) was added and the mixture was reacted for 12 hours at 60°C in a nitrogen atmosphere with stirring. Following the reaction, 1,000 mL of water was added over an hour while stirring in a water bath, the product was left standing for 3 hours, and the aqueous lower layer was removed. The base number was measured, the product was neutralized by adding a quantity of dilute hydrochloric acid calculated for neutralization, the toluene and water were distilled off at 110°C and 6.7 KPa (50 mmHg), and the product was filtered, yielding a crude reaction product. The crude reaction product was melted by heating and then precipitated in petroleum ether by addition with stirring. The precipitate was filtered out and dried under reduced pressure to obtain the targeted product.

### O Measurement of sol-gel transition point

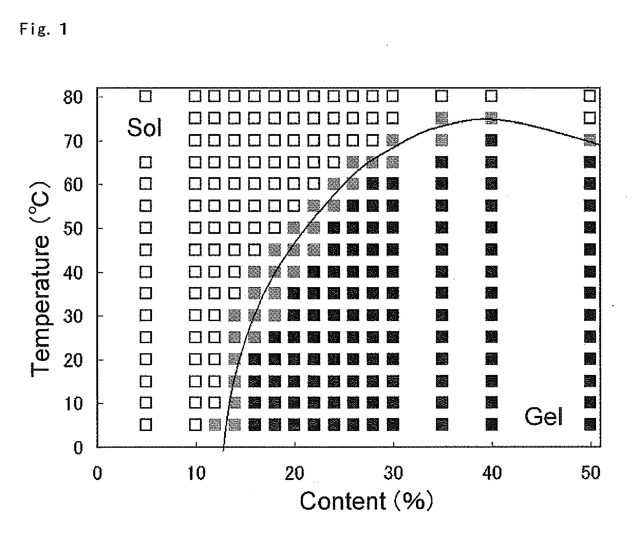

Solutions were prepared using a physiological saline solution in the form of Otsuka normal saline made by Otsuka Pharmaceutical Co., Ltd. ("physiological saline" hereinafter). Fifteen solutions with gel concentrations of 5%, 10%, 12%, 14%, 16%, 18%, 20%, 22%, 24%, 26%, 28%, 30%, 35%, 40%, and 50% were prepared. Samples were prepared by accurately weighing out into 5 mL vials mixtures of mono-PEG and di-PEG with a di-PEG content of 5.4 weight percent so as to achieve the prescribed concentrations, and the physiological saline was added. The solutions that were prepared were heated overnight (about 16 hours) in a DP32 vacuum low-temperature drier made by Yamato Scientific Co., Ltd. set to ordinary pressure and 80°C. The solutions obtained were left standing at room temperature (about 25°C) for three days and then used for measurement. Measurement was conducted using the constant temperature water bath of a BH301 precision low-temperature warm water apparatus made by Yamato Scientific Co., Ltd. by immersing the vial in the water bath for one hour and observing the change in the state thereon. Measurements were made at sixteen temperature points: 5°C, 10°C, 15°C, 20°C, 25°C, 30°C, 35°C, 40°C, 45°C, 50°C, 55°C, 60°C, 65°C, 70°C, 75°C, and 80°C. Measurement was conducted by the method of raising the temperature from a low temperature. The observation method consisted of turning the vial upside down and visually observing the presence or absence of fluidity. The states were then plotted to prepare Fig. 1, showing the sol-gel transition points at various concentrations. In the plot of Fig.1, hollow white represents the sol state, gray represents a mixture of sol and gel, and black represents the gel state.

### ○ Results of measurement of sol-gel transition point

As the concentration increased, the sol-gel transition temperature rose. At a gel concentration of 40 percent, the transition temperature was 75°C, and at temperatures close to that of the human eye (body temperature) -- 35 to 40°Cthe optimal gel concentration for maintaining a gel state was 20 to 22 percent.

**[Table 1]**

| Sample No. | Di-PEG content (mass %) | Gel toughness *1) | Transparency *2) | Fluidity *3) | Time required for gelation (min)*4) | Refractive index (n_{D}: 37°C) | Handling *5) |
|---|---|---|---|---|---|---|---|
| 1 | 4.1 | 2 | ○ | 2 | 40 | 1.3593 | Some difficulty |
| 2 | 5.4 | 3 | ○ | 3 | 30 | 1.3595 | Easy |
| 3 | 6.1 | 3 | ○ | 3 | 30 | 1.3597 | Easy |
| 4 | 8.9 | 4 | ○ | 4 | 30 | 1.3596 | Easy |
| 8 | 8.5 | 4 | ○ | 4 | 20 | 1.3500 | Easy |
| 9 | 10.0 | 3 | ○ | 3 | 40 | 1.3562 | Easy |
| 10 | 20.0 | 4 | ○ | 4 | 30 | 1.3574 | Easy |
| 11 | 30.0 | 4 | ○ | 5 | 40 | 1.3585 | Some difficulty |
| 12 | 40.0 | 4 | ○ | 5 | 40 | 1.3588 | Some difficultly |
| 13 | 50.0 | 5 | ○ | 5 | 30 | 1.3596 | Some difficulty |
| 14 | 100.0 | 5 | ○ | 5 | 5 | 1.3596 | Difficult |

| | | | | | | | |
|---|---|---|---|---|---|---|---|
| *1) The toughness of the gel at 37°C (close to body temperature) was evaluated on a five-step scale 1: Liquid, and thus no tamponade effect. 2: Some fluidity, but in gel form, with tamponade effect anticipated. 3: Low fluidity and gel form, with tamponade effect. 4: Low fluidity and brittleness, but tamponade effect present. 5: Brittleness and toughness present, so no tamponade effect anticipated. *2) The transparency of the gel was visually observed at room temperature and evaluated on a three-step scale: (○: Transparent; Δ: somewhat transparent; X: not transparent). *3) Fluidity was evaluated at 70°C on a five-step scale (packing property in injection device) 1: Flowing 2: Some viscosity, fluidity present 3: Viscosity and fluidity present 4: High viscosity, low fluidity 5: Tough, with no fluidity. *4) Gelation time at 37°C of samples melting at 70°C (samples 1 to 10) and 80°C (Samples 11 to 14) The time required for gelation in the vicinity of body temperature and to permit packing into the injection apparatus *5) Ease of injection with a syringe (21 gauge) (Easy, some difficulty, difficult) | | | | | | | |

### ○ Results of evaluation of physical properties of gels

The physical properties of the gels were evaluated in terms of handling of the gel and the state of toughness in the form of a state with less fluidity than the liquid state permitting the anticipation of a tamponade effect based on change in the di-PEG content (ease of injection), the fluidity at 70°C based on the packing property in the injection apparatus, and the time required for gelation following injection. As the di-PEG content increased, the gel gradually hardened from a state permitting the pulling of filaments. When the di-PEG content exceeded 20 weight percent, the gel became a tough, brittle jelly, and a change in fluidity was confirmed. When 30 weight percent was exceeded, the gel did not melt at 70°C, and the sol-gel transition temperature was determined to rise. At a di-PEG content of 4.1 weight percent, the gel was excessively soft and unsuitable for use as a substitute for ophthalmologic organs such as lenses and the vitreous body. In terms of handling, as well, the fluidity was excessively high and handling was somewhat difficult. Gels with di-PEG contents of 30 and 40 weight percent were tough, brittle jellies in terms of fluidity and handling, and were somewhat difficult to pack into injection apparatus and inject with syringes. However, heating to the sol-gel transition point of about 70°C or higher converted them to sols and permitted ready packing into injection apparatus, with gelation times that were equivalent to those of gels with di-PEG contents of 20 weight percent and lower.

### ○ Light transmittance of gels

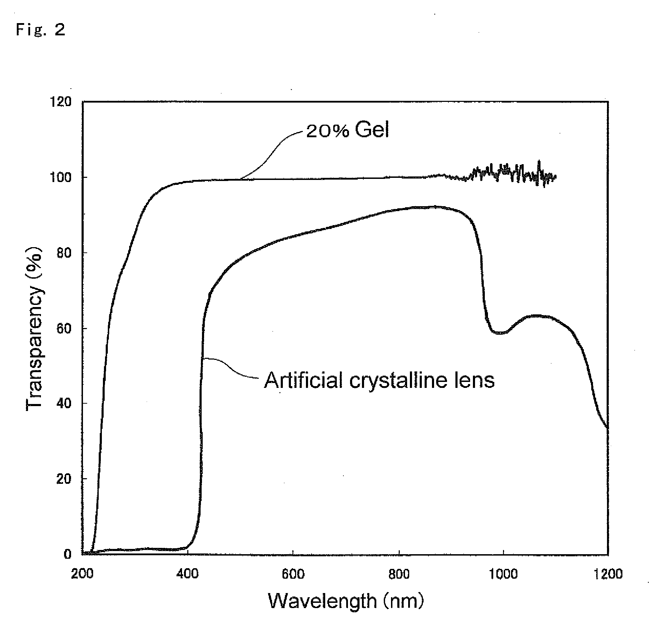

The light transmittance of gels with gel concentrations of 20 percent and a di-PEG content of 5.4 weight percent were measured with a model U-3000 Hitachi self-recording spectrophotometer made by Hitachi Ltd. and compared to the light transmittance curve of the vitreous body of the human eye (Boettner & Wolter, Invest. 0 pH thal. 1.776 (1962)) (see Fig. 2).

In terms of transparency, none of the gels of the various di-PEG contents exhibited clouding. They all had refractive indexes of 1.35 to 1.36, which was not much different from the 1.336 of the vitreous body of a healthy eye. The graph of light transmittance revealed an optical transparency roughly equivalent to that of the human vitreous body, and almost all ultraviolet light was cut, indicating optical characteristics that were entirely adequate for practical use.

### ○ Pathological observation

To confirm the safety of the composition having gelation ability of the present invention on eye tissue, a mono-PEG and di-PEG mixture with a di-PEG content of 5.4 weight percent was prepared with a gel concentration of 20 percent, sterilized for 20 minutes at 121°C in an autoclave, and employed as sample for injection into the eyes of domestic rabbits. The eyes of the domestic rabbits were pathologically observed. The vitreous bodies of five eyes of five colored domestic rabbits were excised and the gel was fully injected with a 21 gauge syringe. At week one, week four, and month three following surgery, the anterior chamber protein concentration, anterior eye part, and the ocular fundus were examined. Electroretinograms were taken prior to surgery and at three months after surgery, and the amplitudes of the a waves and b waves were compared. Three eyes from three colored domestic rabbits from which the vitreous body had been simply excised were employed as controls. The eyeballs were collected at month three following surgery and pathologically examined. The results revealed no abnormality in the anterior eye part or ocular fundus in any of the cases. With the exception that a slight increase in eye pressure was observed in just one eye one week after surgery, the eye pressure remained roughly the same as prior to surgery. A slight rise in the anterior chamber protein concentration was observed in all cases at one week following surgery, but it returned to the pre-surgical level one month following surgery in the same manner as the controls. Comparison of amplitude in the electroretinograms revealed 80 percent or better relative to pre-surgery in all cases, so no drop in retinal function was thought to have occurred. Nothing problematic was found in any of the cases at the junction between the retina and the vitreous body by optical microscopy. Based on the above, the ophthalmologic organ substitute material PEG of the present invention exhibited nothing that would indicate a problem in the pathological observation of domestic rabbit eyes, had no cell toxicity in eye tissue, and did not affect retinal function.

### [Industrial Applicability]

The ophthalmologic composition of the present invention is not toxic to cells, is transparent, has a stable structure, is injected and removed rapidly by changing the temperature, has physical properties suited to eye tissue with a tamponade effect, and is useful as a material for replacing ophthalmologic organs.

## Claims

1. An ophthalmologic composition having gelation ability, comprising the polyethylene glycol denoted by Formula 1, one end of which is modified with a long-chain alkyl group; the polyethylene glycol denoted by Formula 2, both ends of which are modified with long-chain alkyl groups; and a solvent:
R₁-O-(CH₂CH₂O-)ₙ₁-R₂ (Formula 1)
wherein
R₁ denotes an alkyl group with 1 to 4 carbon atoms;
R₂ denotes an alkyl group with 16 to 22 carbon atoms, and
the average number of moles of added oxyethylene groups n1 falls within a range of 45 to 450;
R₃-O-(-CH₂CH₂O-)ₙ₂-R₄ (Formula 2)
wherein
each of R₃ and R₄ independently denotes an alkyl group with 16 to 22 carbon atoms, and
the average number of moles of added oxyethylene groups n2 falls within a range of 45 to 450.

2. The composition according to claim 1, wherein the mass ratio of the polyethylene glycol denoted by Formula 1 and the polyethylene glycol denoted by Formula 2 is in a range of 50 to 99:50 to 1.

3. The composition according to claim 1 or 2, wherein the solvent is water or physiological saline.

4. The composition according to any one of claims 1 to 3, wherein the combined content of the polyethylene glycol denoted by Formula 1 and the polyethylene glycol denoted by Formula 2 is in a range of 10 to 40 weight percent.

5. The composition according to any one of claims 1 to 4, wherein a pH-adjusting agent is further incorporated.

6. The composition according to any one of claims 1 to 5, wherein a fluorescent dye and/or coloring agent is further incorporated.

7. The composition according to any one of claims 1 to 6, wherein the composition is employed as an artificial vitreous body.

8. The composition according to any one of claims 1 to 6, wherein the composition is employed as an artificial crystalline lens.
